(19) Europäisches Patentamt
European Patent Office
Office européen des brevets

(11) Veröffentlichungsnummer: **0 037 934**
**A2**

(12) # EUROPÄISCHE PATENTANMELDUNG

(21) Anmeldenummer: **81102233.4**

(22) Anmeldetag: **25.03.81**

(51) Int. Cl.³: **C 07 D 217/18**, C 07 D 401/12, C 07 D 405/12, C 07 D 471/10, C 07 D 491/04, A 61 K 31/47 // (C07D471/10, 241/00, 221/00),(C07D491/04, 317/00, 221/00)

(30) Priorität: **11.04.80 DE 3013906**

(43) Veröffentlichungstag der Anmeldung: **21.10.81 Patentblatt 81/42**

(84) Benannte Vertragsstaaten: **AT BE CH DE FR IT LI LU NL SE**

(71) Anmelder: **C.H. BOEHRINGER SOHN, Postfach 200, D-6507 Ingelheim am Rhein (DE)**

(72) Erfinder: **Lösel, Walter, Dr., Im Herzenacker 26, D-6535 Gau-Algesheim (DE)**
Erfinder: **Esser, Franz, Dr., Albrecht-Dürer-Strasse 24, D-6507 Ingelheim/Rhein (DE)**
Erfinder: **Roos, Otto, Dr., Elsheimer Strasse 36, D-6501 Schwabenheim (DE)**
Erfinder: **Reichl, Richard, Dr., Im Hippel 55, D-6535 Gau-Algesheim (DE)**
Erfinder: **Kuhn, Franz Josef, Dr., Melchiorstrasse 6, D-6530 Bingen/Rhein (DE)**
Erfinder: **Traunecker, Werner, Dr., Birkenweg 1, D-6531 Münster-Sarmsheim (DE)**

(54) **Substituierte alpha-Aminocarbonyl-1-benzyl-3,4-dihydro-isochinoline, Verfahren zu deren Herstellung und deren Verwendung.**

(57) Die Erfindung betrifft neue substituierte $\alpha$-Aminocarbonyl-1-benzyl-3,4-dihydro-isochinoline der allgemeinen Formel I

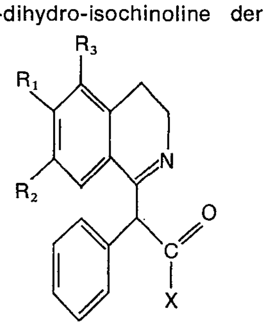

I

worin

$R_1$, $R_2$, $R_3$ Wasserstoff, Hydroxy-, Methoxy- oder kombiniert Methylendioxygruppen,

X Dialkylaminogruppen mit 1–3 Kohlenstoffatomen oder heterocyclische Reste wie Aziridinyl-, Azetidinyl oder Pyrrolidinylgruppen; Morpholino- oder N'-Methyl- bzw. N'-Benzylpiperazinoreste; oder

NHR worin

R niedere Alkylgruppen mit 1–4 Kohlenstoffatomen, Alkenyl- oder Alkinylgruppen mit 3 Kohlenstoffatomen, Cycloalkylgruppen mit 3–6 Kohlenstoffatomen, Morpholino- oder Pyridylgruppen oder Di- bzw. Tri-Methylenreste substituiert durch eine Hydroxy-Methoxy-, Dimethylamino-, Phenyl-, Methoxyphenyl-, Dimethoxyphenyl-, Methylendioxyphenyl-, Morpholino- oder Indolylgruppen bedeuten, in racemischer oder optisch aktiver Form sowie deren Säureadditionssalze.

Die neuen Verbindungen können z.B. durch Cyclisierung eines (gemischten) Diamids der Phenylmalonsäure mit der allgemeinen Formel II

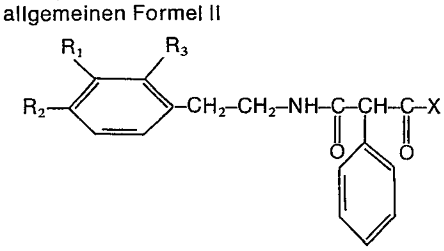

II

worin $R_1$, $R_2$, $R_3$ und X die oben angegebenen Bedeutungen haben, in Gegenwart eines Kondensationsmittels, wie Lewissäuren oder starke Mineralsäuren und gegebenenfalls Umsetzung der erhaltenen freien Base mit einer geeigneten Säure zum Säureadditionssalz hergestellt werden.

Die neuen Verbindungen sind zur Verbesserung der Gewebsdurchblutung und Gewebssauerstoffversorgung, insbesondere im Zentralnervensystem verwendbar, ferner haben sie eine kontraktilitätssteigernde und blutdruckbeeinflussende Komponente.

EP 0 037 934 A2

ACTORUM AG

COMPLETE DOCUMENT

Gegenstand der Anmeldung sind substituierte α-Aminocarbonyl-1-benzyl-3,4-dihydro-isochinoline der allgemeinen Formel

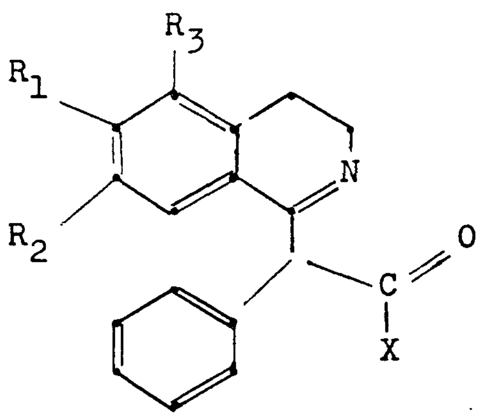

I

worin

$R_1$, $R_2$, $R_3$ Wasserstoff, Hydroxy-, Methoxy- oder kombiniert Methylendioxygruppen,

X Dialkylaminogruppen mit 1 - 3 Kohlenstoffatomen oder heteroyclische Reste wie Aziridinyl-, Azetidinyl oder Pyrrolidinylgruppen; Morpholino- oder N'-Methyl- bzw. N'-Benzylpiperazinoreste; oder

NHR worin

R niedere Alkylgruppen mit 1 - 4 Kohlenstoffatomen, Alkenyl- oder Alkinylgruppen mit 3 Kohlenstoffatomen, Cycloalkylgruppen mit 3 - 6 Kohlenstoffatomen, Morpholino- oder oder Pyridylgruppen oder Di- bzw. Tri-Methylenreste substituiert durch eine Hydroxy-, Methoxy-, Dimethylamino-, Phenyl-Methoxyphenyl-, Dimethoxyphenyl-,

Methylendioxyphenyl-, Morpholino- oder Indolylgruppen bedeuten, in racemischer oder optisch aktiver Form sowie deren Säureadditonssalze mit einem physiologisch geeigneten Anion.

Als Säureadditionssalze können beliebige physiologisch unbedenkliche, mit anorganischen oder organischen Säuren gebildete Salze, z.B. Hydrochloride, Hydrogensulfate, (Hydrogen-)phosphate, Tartrate, Succinate, Maleate, Benzoate, Acetate, Propionate, Lactate, Ascorbinate, etc. hergestellt werden.

Die Darstellung der erfindungsgemäßen Verbindungen geschieht in der Weise, daß man ein (gemischtes) Diamid der Phenylmalonsäure, das durch die allgemeine Formel II

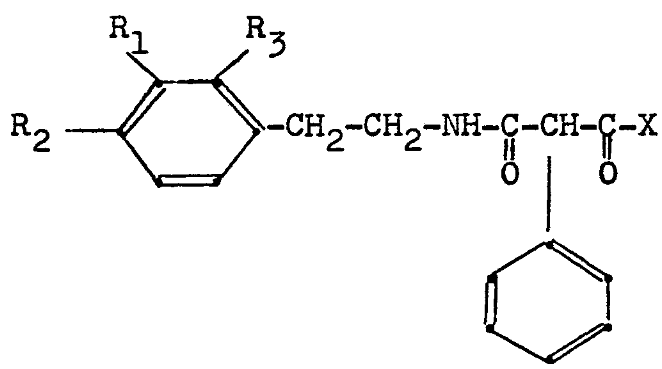

II

beschrieben ist, und in der die Substituenten $R_1$, $R_2$, $R_3$ und X die bereits oben angegebenen Bedeutungen haben, in Gegenwart eines geeigneten Kondensationsmittels in an sich bekannter Weise zum entsprechenden 3,4-Dihydroisochinolin cyclisiert und, gegebenenfalls anschließend ein erhaltenes Salz in die freie Base überführt und/oder die erhaltene freie Base mit einer physiologisch verträglichen Säure in ihr Salz.

Als Kondendationsmittel eignen sich zahlreiche Lewissäuren, wie z.B. Phosphoroxichlorid, Bortrifluorid, Zinntetrachlorid oder Titantetrachlorid, aber auch

starke Mineralsäuren wie Schwefelsäure, Fluorsulfonsäuren, Fluorwasserstoffsäure oder Polyphosphorsäure. Sie werden gewöhnlich im Überschuß eingesetzt. Bevorzugt wird Phosphoroxichlorid.

Die Cyclisierungsreaktion kann in Gegenwart oder in Abwesenheit eines Lösungsmittels durchgeführt werden. Geeignet sind alle inerten Lösungsmittel, soweit sie eine ausreichende Löslichkeit für die Reaktionspartner besitzen und einen ausreichend hohen Siedepunkt aufweisen. Beispiele sind Benzol, Alkylbenzole, Chlorbenzole, Dekalin, Chloroform, Methylenchlorid, Acetonitril und dergl. Eine Variante des Verfahrens besteht darin, das Kondensationsmittel, beispielsweise Phosphoroxichlorid, selbst als Lösungsmittel zu verwenden.

Bezüglich der Reaktionstemperatur bestehen keine speziellen Bedingungen. Die erfindungsgemäße Umsetzung kann innerhalb eines großen Temperaturbereiches, vorzugsweise unter Erwärmen oder Erhitzen bis etwa zum Siedepunkt des Lösungsmittels durchgeführt werden.

Die Ausgangsverbindungen der Formel II stellen zum Teil neue Verbindungen dar und wurden nach folgendem Schema hergestellt

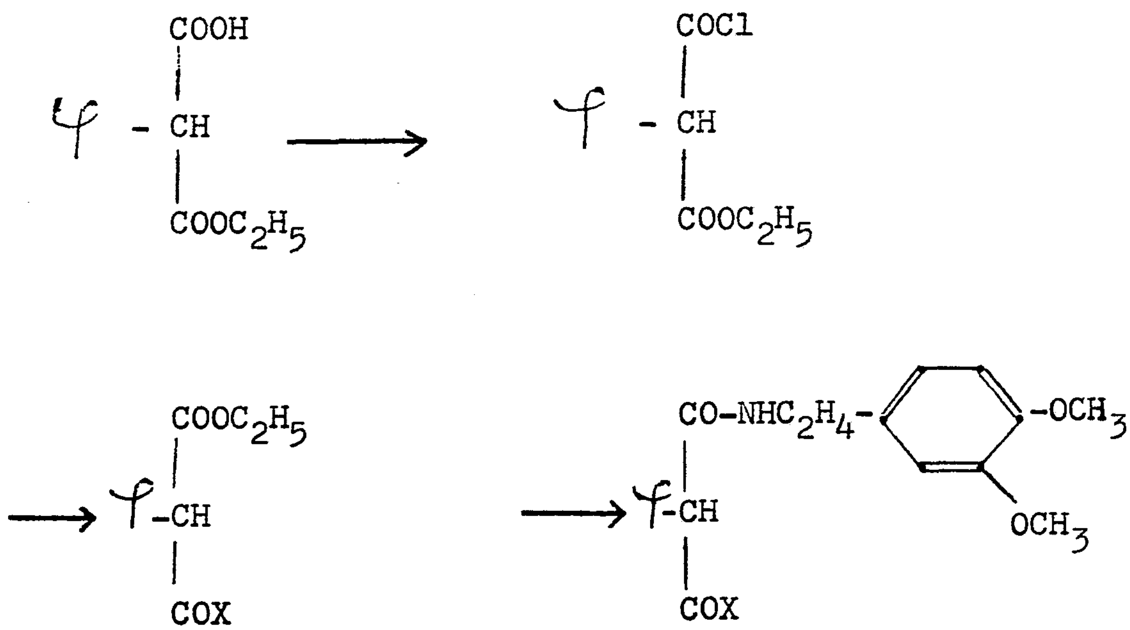

II

Als Eingangsstufe werden Phenylmalonsäure mit Thionylchlorid behandelt und weiterführend mit einem Amin HX umgesetzt. Das Ausgangsprodukt der Formel II erreicht man dann durch Umsetzung mit 2-(3', 4'-Dimethoxyphenyl)-äthylamin unter Stickstoffatmosphäre.

Die erfindungsgemäßen Verbindungen verbessern die Gewebsdurchblutung und Gewebssauerstoffversorgung, besonders im Zentralnervensystem. Zudem verfügen sie über eine kontraktilitätssteigernde und blutdruckbeeinflussende Wirkkomponente.

Besonders wirksam sind Verbindungen, worin X ein ringschlüssiges, heterocyclisches System oder ein Dialkylamin darstellt.

Die Verbindungen der allgemeinen Formel I in racemischer oder optisch aktiver Form sowie deren Säureadditonssalze können auch in Kombination mit andersartigen Wirkstoffen eingesetzt werden. Geeignete galenische Darreichungsformen sind beispielsweise Tabletten, Kapseln, Zäpfchen, Lösungen oder Pulver; hierbei können zu deren Herstellung die üblicherweise verwendeten galenischen Hilfs-, Träger-, Spreng- oder Schmiermittel bzw. Substanzen zur Erzielung einer Depotwirkung angewendet werden.

Die erfindungsgemäßen Verbindungen haben eine signifikant längere Wirkungsdauer als handelsübliche Produkte. Im Vergleich zu Xanthinol-nicotinat liegt die Überlegenheit bei 60 %, bezogen auf die gesteigerte Hindurchblutung am Katzenmodell.

Aus der Hemmung eines Thiopentalschadens an der Rattenpfote ergibt sich mit der genannten Vergleichssubstanz für die durchblutungsfördernde und kreislauf-tonisierende Wirkung eine Dosisrelation von 1 : 100. Für den Resorptionskoeffizienten - also die Relation perorale durch intravenöse Dosierung - besitzen die erfindungsgemäßen Verbindungen eine Überlegenheit von 2 : 1. Als Dosierung wird ein Bereich zwischen 1 - 50 mg/kg, vorzugsweise 10 - 40 mg/kg (peroral) oder 0,1 - 10 mg/kg, vorzugsweise 2 - 7 mg/kg (intravenös) empfohlen.

Folgende Beispiele erläutern die Erfindung, ohne sie in ihrem Umfang zu beschränken.

## Vorprodukte

### Beispiel a

### Phenylmalonsäuremonoäthylesterchlorid

105 g (0,5 Mol) Phenylmalonsäuremonoäthylester (JACS 74, 5897 (1952) werden zusammen mit 119 g Thionylchlorid im Wasserbad 90 Min. unter Rühren und Rückfluß erhitzt. Überschüssiges Thionylchlorid wird im Vakuum abgezogen, der Rückstand unter vermindertem Druck (130 - 135°/15 mm) destilliert, wobei 85,0 g (= 75 % d.Theorie) Phenylmalonsäuremonoäthylesterchlorid erhalten werden.

### Beispiel b

### Phenylmalonsäuremonoäthylester-2-(3',4'-dimethoxyphenyl)-äthylamid

295 g (1,3 Mol) Phenylmalonsäuremonoäthylesterchlorid in 500 ml abs. Tetrahydrofuran werden bei Raumtemperatur unter Rühren und $N_2$-Schutz in eine gekühlte Mischung von 236 g (1,3 Mol) 2-(3',4'-Dimethoxyphenyl)-äthylamin, 171 g Triäthylamin und 500 ml abs. Tetrahydrofuran eingetropft. Nach beendeter Umsetzung wird ausgefallenes Triäthylammoniumchlorid abgesaugt, das Filtrat eingeengt, der feste Rückstand zwischen Wasser und Methylenchlorid verteilt. Die organische Phase wird über $Na_2SO_4$ getrocknet. Nach dem Abdampfen des Lösungsmittels wird aus Essigester umkristallisiert.

Fp.: 103°, Ausbeute: 342 g (= 71 % d. Theorie).

Beispiel c

α-Diäthylaminocarbonyl-phenylessigsäure-2-(3', 4'-dimethoxyphenyl)-äthylamid

16,7 g Phenylmalonsäuremonoäthylester-2-(3',4'-dimethoxyphenyl)-äthylamid und 120 ml Diäthylamin werden im Autoklaven 48 Stunden auf 120 - 130° erhitzt. Das Reaktionsgemisch wird mit 500 ml Äthanol versetzt und bei Siedehitze mit Aktivkohle behandelt. Nach dem Abkühlen wird abgesaugt, eingedampft und der Rückstand aus Essigester-Petroläther 40 - 80° kristallisiert, wenn nötig, wird zuvor über eine Kieselgelsäule ($CH_2Cl_2$:MeOH = 100 : 1 ⟶ 100 : 2) gereinigt. Fp.: 81 - 85°;
Ausbeute: 14 g (= 78 % d.Theorie)

Beispiel 1

α-Diäthylaminocarbonyl-1-benzyl-6,7-dimethoxy-3,4-dihydro-isochinolin-hydrochlorid

33 g α-Diäthylaminocarbonyl-phenylessigsäure-2-(3',4'-dimethoxyphenyl)-äthylamid werden in 120 ml Acetonitril gelöst und mit 18 ml Phosphoroxichlorid versetzt. Das Reaktionsgemisch wird ca. 2 Stunden auf Rückflußtemperatur erhitzt. Danach wird eingeengt, der Rückstand in 200 ml Methylenchlorid aufgenommen und durch Einrühren in eine Eiswasser-Pottasche-Lösung alkalisch gestellt. Nach der üblichen Aufarbeitung - ausschütteln mit Methylenchlorid, trocknen der organischen Phase über $Na_2SO_4$, abziehen des Lösungsmittels etc. wird über eine Kieselgelsäule ($CH_2Cl_2$/MeOH = 100/1 ⟶ 100/2 gereinigt. Fp.: 170°; Ausbeute 26 g.

Zur Darstellung des Hydrochlorids wrden 26 g Base in möglichst wenig Äthanol gelöst und mit äthanolischer Salzsäure versetzt. Das Hydrochlorid wird durch tropfenweisen Zusatz von abs. Äther und Petroläther 40 : 80° (1:1) zur Kristallisisation gebracht.

Fp.: 203 - 205°; Ausbeute: 17 g (= 49,2 % d.Theorie)

Beispiel 2

1-(α-Dimethylaminocarbonyl-benzyl)-6-methoxy-3,4-dihydro-isochinolin-hydrochlorid

3,9 g (11,5 mMol) Phenylmalonsäure-dimethylamid-[2-(3-methoxyphenyl)-äthylamid] löste man in 60 ml Chloroform, versetzte mit 13,7 g (90 mMol) $POCl_3$ und kochte 6 Stunden am Rückfluß. Das Lösungsmittel wurde am Rotationsverdampfer abgezogen, der Rückstand in $CH_2Cl_2$ gelöst und in eine kalte, gesättigte $K_2CO_3$-Lösung eingetropft. Nach dreimaliger Extraktion mit $CH_2Cl_2$ wurden

die vereinigten organischen Extrakte dreimal mit 2 n HCl extrahiert. Die vereinigten HCl-Phasen wurden mit $K_2CO_3$ alkalisch gestellt und wiederum mit $CH_2Cl_2$ ausgeschüttelt. Die organische Phase wurde getrocknet, unter vermindertem Druck eingeengt und der Rückstand mit ätherischer HCl behandelt. Das ausgefallene Hydrochlorid wurde in Isopropanol gelöst, mit Aktivkohle behandelt und so viel Diäthyläther zugegeben, bis gerade eine Trübung entstand. Über Nacht im Kühlschrank kristallisierte die gewünschte Verbindung als Hydrochlorid.
Ausbeute: 2,3 g (56 % d. Theorie).
Fp.: $124^{\circ}$ C

In analoger Form wurden die folgenden Verbindungen hergestellt:

Tabelle 1

$R_1 = R_2$ = Methoxy, $R_3$ Wasserstoff

| Nr. | X | Salzform | Fp (°C) |
|---|---|---|---|
| 1. | $-NHCH_3$ | Hydrochlorid | 220 |
| 2. | $-N(CH_3)_2$ | Hydrochlorid | 153-155 |
| 3. | $-NHC_2H_5$ | Base | 155-157 |
| 4. | $-N(C_2H_5)_2$ | Hydrochlorid | 190 |
| 5. | $-NH-CH_2-CH_2-OH$ | Hydrochlorid | 212 |
| 6. | $-NH-CH_2-CH_2-OCH_3$ | Base | 107 |
| 7. | $-NH-CH_2-CH_2-CH_2-OCH_3$ | Hydrochlorid | 164 |
| 8. | $-NH-CH_2-CH(CH_3)_2$ | Hydrochlorid | 214 |
| 9. | $-NH-CH_2-CH_2-CH_2-N(CH_3)_2$ | Dihydrochlorid | 224-226 |
| 10. | 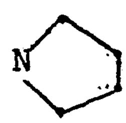 | Hydrochlorid | 157-159 |
| 11. | 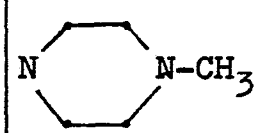 | Base | 78-80 |
| 12. | 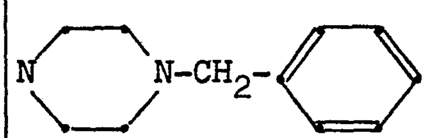 | Dihydrochlorid | 226-229 |
| 13. | $-NH-CH_2-CH_2-$ 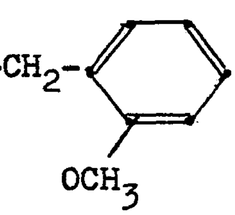 | Base | 140 |
| 14. | $-NH-CH_2-CH_2-$ 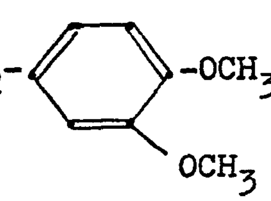 | Hydrochlorid | 161 |

| Nr. | X | Salzform | Fp.(°C) |
|---|---|---|---|
| 15. | -NH-$CH_2$-$CH_2$- 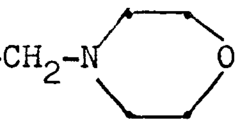 | Base | 152 |
| 16. | -NH-$CH_2$-$CH_2$ 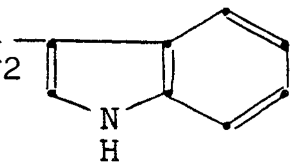 | Base | 163 |
| 17. | -NH- 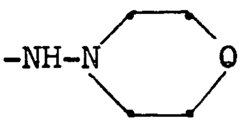 | Base | 193-195 |
| 18. | -NH- 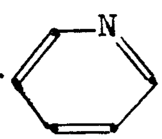 | Hydrochlorid | 197-202 |
| 19. | -NH-$CH_2$-$CH_2$- 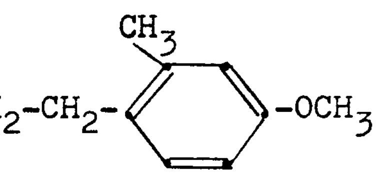 | Base | 168-170 |
| 20. | -NH-$CH_2$-$CH_2$- 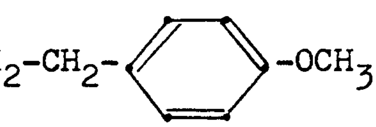 | Base | 123-125 |
| 21. | -NH-$CH_2$-$CH_2$- 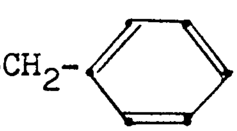 | Hydrochlorid | 109 |

0037934

| Nr. | X | Salzform | Fp.(° C) |
|---|---|---|---|
| 22. | -NH- 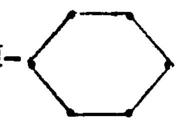 | Base | 130-135 |
| 23. | $-NH-CH_2-CH_2-$ 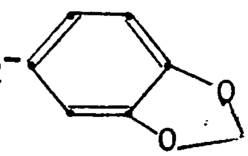 | Base | 118-120 |
| 24. | $-NH-CH_2-CH_2-CH_3$ | Hydrochlorid | 71-78 |
| 25. | $NH-CH_2-CH_2-CH_2-CH_3$ | Hydrochlorid | 79-81 |
| 26. | $NH-CH_2-CH=CH_2$ | Hydrochlorid | 205-207 |
| 27. | $NH-CH_2-C \equiv CH$ | Hydrochlorid | 173-175 |
| 28. | NH- 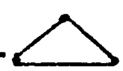 | Hydrochlorid | 227-228 |
| 29. | $NH-CH\ (CH_3)_2$ | Hydrochlorid | 226-227 |
| 30. | $NH-C\ (CH_3)_3$ | Hydrochlorid | 225-226 |
| 31. | $NH-CH_2-CH_2-CH_2-CH_2-CH_3$ | Hydrochlorid | 88-94 |
| 32. | $NH-CH(CH_3)-CH_2-CH_3$ | Hydrochlorid | 214-215 |
| 33. | $NH-CH_2-CH_2-CH(CH_3)_2$ | Hydrochlorid | 90-93 |
| 34. | $NH-CH_2-CH(OH)-CH_3$ | Hydrochlorid | 201-202 |

| Nr. | X | Salzform | Fp.(° C) |
|---|---|---|---|
| 35. | $NH-CH_2-CH_2-N(CH_3)_2$ | Dihydrochlorid | 134-143 |
| 36. | $NH-CH_2-CH_2-$ 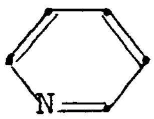 | Base | 110-112 |
| 37. | $NH-CH_2-CH_2-$ 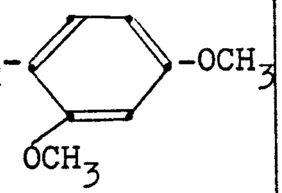 | Base | 168-170 |
| 38. | $N-(CH_2-CH_2-$ 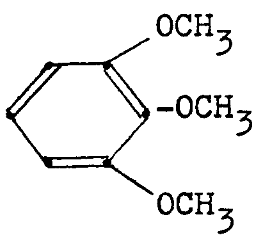 $)_2$ | Hydrochlorid | 56-64 amorph |
| 39. | 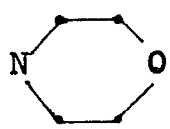 | Hydrochlorid | 59-68 amorph |
| 40. | 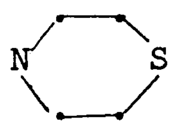 | Hydrochlorid | 195-197 |
| 41. | 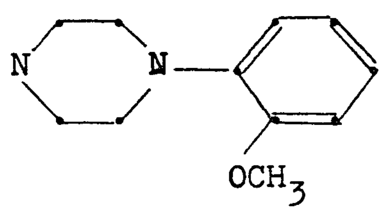 | Hydrochlorid | 183 |
| 42. | $NH-CH_2-$ 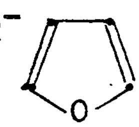 | Base | 80-83 |

Tabelle 2

$R_1$, $R_2$ = Methylendioxy, $R_3$ Wasserstoff

| Nr. | X | Salzform | Fp.(° C) |
|---|---|---|---|
| 43. | $-NH-CH_2-CH_2-$ 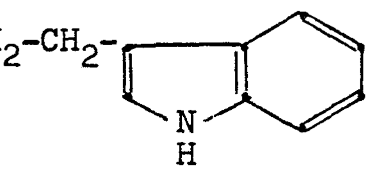 | Base | 99-90 |
| 44. | $-NH-CH_2-CH_2-$ 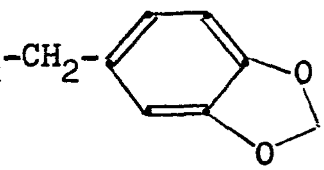 | Hydrochlorid | 115-120 |
| 45. | $NH-CH_2-CH_2-$ 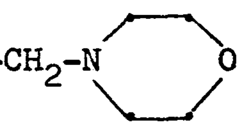 | Base | 155-156 |
| 46. | 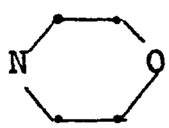 | Base | 165 |
| 47. | 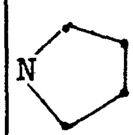 | Base | 199 |

Tabelle 3

$R_1$ = Methoxy, $R_2$ = Hydroxy, $R_3$ Wasserstoff

| Nr. | X | Salzform | Fp.($^oC$) |
|---|---|---|---|
| 48. | $-NH-CH_2-CH_2-$ 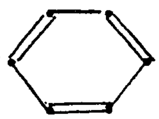 | Hydrochlorid | 108-112 |
| 49. | $-NH-CH_2-CH(CH_3)_2$ | Base | 154-158 |
| 50. | 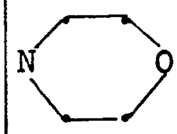 | Hydrochlorid | 158-163 |
| 51. | $NH-CH_2-CH_2-$ 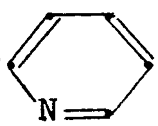 | Base | 143-146 |
| 52. | $NH-CH_2-CH_2-$ 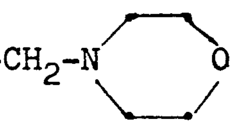 | Base | 216-218 |
| 53. | NH- 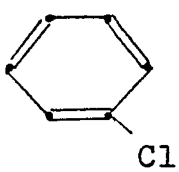 | Base | 148-153 |
| 54. | NH- 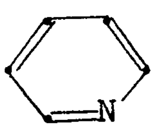 | Hydrochlorid | 177-183 |
| 55. | $NH-CH_2-CH_2-$ 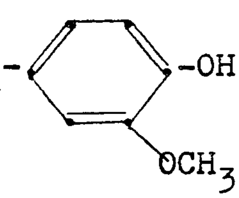 | Hydrochlorid | 136 |

| Nr. | X | Salzform | Fp.(°C) |
|---|---|---|---|
| 56. | 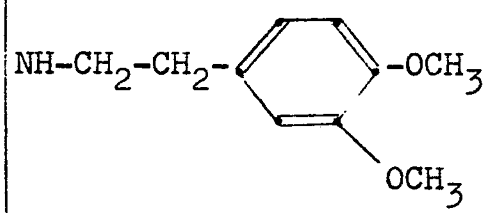 | Hydrochlorid | 161-165 |
| 57. | 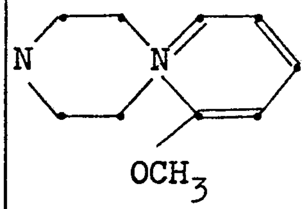 | Hydrochlorid | 198 |
| 58. | 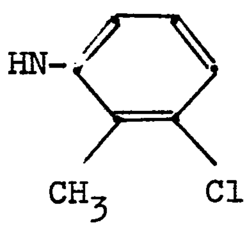 | Hydrochlorid | 167 |
| 59. | 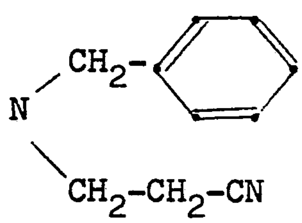 | Hydrochlorid | 167-172 |
| 60. | $NH-CH_2-CH_2-CH_2-CH_2-CH_3$ | Base | 149 |
| 61. | $NH-CH_2-CH_2-CH_2-CH_3$ | Base | 147 |
| 62. | $NH-CH_2-CH=CH_2$ | Base | 167-169 |
| 63. | $NH-CH(CH_3)_2$ | Hydrochlorid | 152 |
| 64. | 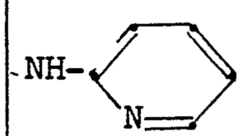 | Base | 129-131 |

Tabelle 4

$R_1$ = Hydroxy, $R_2$ = Methoxy, $R_3$ Wasserstoff

| Nr. | X | Salzform | Fp.(°C) |
|---|---|---|---|
| 65. | $N(C_2H_5)_2$ | Hydrochlorid | 199°C |

Tabelle 5

$R_1$ = Methoxy, $R_2$ = $R_3$ = Wasserstoff

| Nr. | X | Salzform | Fp.(°C) |
|---|---|---|---|
| 66. | NH-$C_2H_5$ | Hydrochlorid | 158 |
| 67. | NH-$CH_3$ | Hydrochlorid | 155 |
| 68. | NH-$CH_2$-$CH_2$- 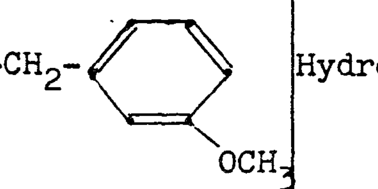 | Hydrochlorid | 104 (Z) |
| 69. | $N(CH_3)C_2H_5$ | Hydrochlorid | ~60° hygrosk. |
| 70. | NH-$CH_2$- 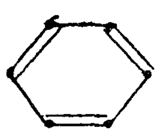 | Hydrochlorid | ~ 120° |

Tabelle 6

$R_1 = R_2 = R_3 =$ Methoxy

| Nr. | X | Salzform | Fp.($^oC$) |
| --- | --- | --- | --- |
| 71 | $N(CH_3)C_2H_5$ | Toluolsulfonat | hygrosk. |
| 72 | $NH-CH_2-$ 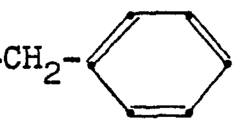 | Hydrochlorid | 180 |
| 73 | $NH-CH_2-CH_2-$ 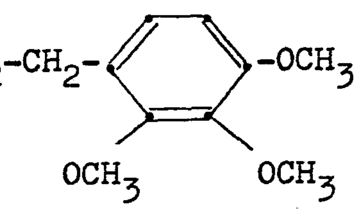 | Hydrochlorid | 89 (Z) |
| 74 | NH- 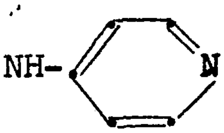 | Base | 117-120° |

## Formulierungsbeispiele

### Beispiel 1: Tabletten

| | |
|---|---|
| Wirkstoff gemäß Erfindung | 40,0 mg |
| Milchzurcker | 100,0 mg |
| Maisstärke | 50,0 mg |
| kolloidale Kieselsäure | 2,0 mg |
| Magnesiumstearat | 3,0 mg |
| insagesamt | 200,0 mg |

Herstellung:

Der Wirkstoff wird mit einem Teil der Hilfsstoffe gemischt und mit einer Lösung der löslichen Stärke in Wasser granuliert. Nach dem Trocknen des Granulats wird der Rest der Hilfsstoffe zugemischt und die Mischung zu Tabletten verpreßt.

### Beispiel 2: Dragées

| | |
|---|---|
| Wirkstoff gemäß Erfindung | 20,0 mg |
| Milchzucker | 100,0 mg |
| Maisstärke | 65,0 mg |
| kolloidale Kieselsäure | 2,0 mg |
| lösliche Stärke | 5,0 mg |
| Magnesiumstearat | 3,0 mg |
| insgesamt | 195,0 mg |

Herstellung:

Der Wirkstoff und die Hilfsstoffe werden, wie in Beispiel 1 beschrieben, zu Tablettenkernen verpreßt, die mit Zucker, Talcum und Gummi arabicum in üblicher Weise dragiert werden.

Beispiel 3: Suppositorien

| | |
|---|---|
| Wirkstoff gemäß Erfindung | 50,0 mg |
| Milchzucker | 250,0 mg |
| Suppositorienmasse q.s.ad | 1,7 g |

Herstellung:

Der Wirkstoff und der Milchzucker werden miteinander vermischt und die Mischung in der geschmolzenen Suppositorienmasse gleichmäßig suspendiert. Die Suspensionen werden in gekühlte Formen zu Suppositorien von 1,7 g Gewicht ausgegossen.

Beispiel 4: Ampullen

| | |
|---|---|
| Wirkstoff gemäß Erfindung | 20,0 mg |
| Natriumchlorid | 5,0 mg |
| Bi-destilliertes Wasser q. s. ad | 2,0 ml |

Herstellung:

Der Wirkstoff und das Natriumchlorid werden in bidestilliertem Wasser gelöst und die Lösung in Ampullen steril abgefüllt.

Beispiel 5: Ampullen

| | |
|---|---|
| Wirkstoff gemäß Erfindung | 10,0 mg |
| Natriumchlorid | 7,0 mg |
| Bi-destilliertes Wasser q. s. ad | 1,0 mg |

Beispiel 6: Tropfen

| | |
|---|---|
| Wirkstoff gemäß Erfindung | 0,70 g |
| p-Hydroxybenzoesäuremethylester | 0,07 g |
| p-Hydroxybenzoesäureppropylester | 0,03 g |
| Entmineralisiertes Wasser q.s. ad | 100,00 ml |

Herstellung:

Der Wirkstoff und die Konservierungsmittel werden in entmineralisiertem Wasser gelöst, die Lösung filtriert und in Flaschen zu je 100 ml abgefüllt.

# Patentansprüche

1. Verbindungen der allgemeinen Formel I

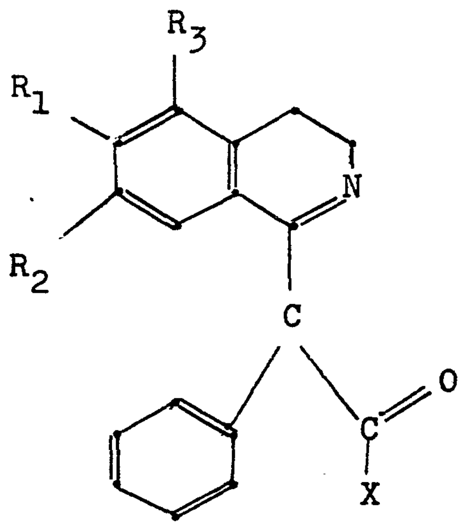

I

worin

$R_1, R_2, R_3$ Wasserstoff, Hydroxy-, Methoxy- oder kombiniert Methylendioxygruppen,

X Dialkylaminogruppen mit 1 - 3 Kohlenstoffatomen oder heterocyclische Reste wie Aziridinyl-, Azetidinyl- oder Pyrrolidinylgruppen; Morpholino- oder N'-Methyl- bzw. N'-Benzylpiperazinoreste; oder

NHR worin

R niedere Alkylgruppen mit 1 - 4 Kohlenstoffatomen, Alkenyl- oder Alkinylgruppen mit 3 Kohlenstoffatomen, Cycloalkylgruppen mit 3 - 6 Kohlenstoffatomen, Morpholino- oder Pyridylgruppen oder Di- bzw. Tri-Methylenreste substituiert durch eine Hydroxy-, Methoxy-, Dimethylamino-, Phenyl-, Methoxyphenyl-, Dimethoxyphenyl-, Methylendioxyphenyl-, Morpholino- oder Indolylgruppe bedeuten; in racemischer oder optisch aktiver Form sowie deren Säureadditionssalze mit einem physiologisch geeigneten Anion.

2. Verbindungen nach Anspruch 1, worin
$R_1$, $R_2$, $R_3$ Wasserstoff, Methoxy- oder Methylendioxygruppen und

X Dialkylaminogruppen mit 1 - 3 Kohlenstoffatomen oder heterocyclische Reste wie Aziridinyl-, Acetidinyl- oder Pyridinylgruppen, einen Morpholino- oder N'-Methyl- bzw. N'-Benzylpiperazinorest in optisch aktiver oder racemischer Form, sowie deren Säureadditionssalze mit einem physiologisch geeigneten Anion.

3. α-Diäthylaminocarbonyl-1-benzyl-6,7-dimethoxy-3,4-dihydroisochinolin in optisch aktiver oder racemischer Form sowie deren Säureadditionssalze mit einem physiologisch geeigneten Anion.

4. α-Hydroxyäthylaminocarbonyl-1-benzyl-6,7-dimethoxy-3,4-dihydroxyisochinolin-hydrochlorid in optisch aktiver oder racemischer Form sowie deren Säureadditionssalze mit einem physiologisch geeigneten Anion.

5. Verfahren zur Herstellung von Verbindungen der allgemeinen Formel I

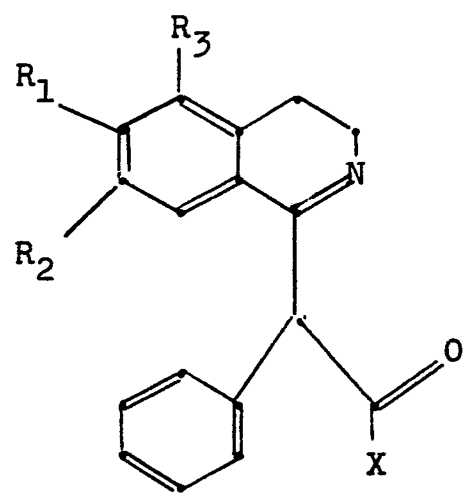

I

worin $R_1$, $R_2$, $R_3$ und X die oben genannte Bedeutung haben, dadurch gekennzeichnet, daß man ein Diamid der Phenylmalonsäure der allgemeinen Formel II

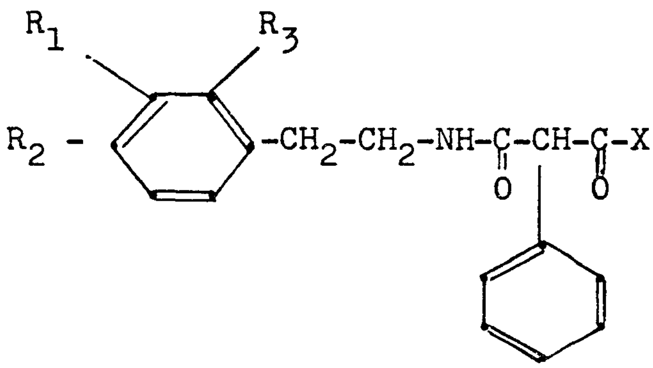

II

mit den oben genannten Bedeutungen für $R_1$, $R_2$, $R_3$ und X in an sich bekannter Weise cyclisiert und gegebenenfalls anschließend ein erhaltenes Salz in die freie Base überführt und/oder die erhaltene freie Base mit einer physiologisch verträglichen Säure in ihr Salz.

6. Verfahren nach Anspruch 5, dadurch gekennzeichnet, daß die Endprodukte der Formel I nach an sich bekannten Methoden in die optischen Antipoden aufgespalten werden.

7. Arzneimittel, bestehend aus einer Verbindung gemäß Ananspruch 1 und üblichen galenischen Hilfs- und Trägerstoffen.

8. Verfahren zur Herstellung eines Arzneimittels gemäß Anspruch 7, dadurch gekennzeichnet, daß man eine Verbindung der allgemeinen Formel I mit üblichen galenischen Hilfs- und Trägerstoffen mischt und in eine dosierbare Form bringt.

9. Methode zur Durchblutungsförderung und Kreislauftonisierung durch Verabreichung eines Arzneimittels gemäß Anspruch 7.